# EUROPEAN PATENT SPECIFICATION

(11) **EP 1 296 716 B1**
(45) Date of publication and mention of the grant of the patent: **08.11.2006**
(21) Application number: 01941409.3
(22) Date of filing: 15.06.2001
(51) Int. Cl.: A61K 45/00, A61K 45/06, A61K 31/195, A61K 31/505, A61K 31/40, A61P 31/06, A61P 9/10

(54) **USE OF A BETA-BLOCKER FOR THE TREATMENT OF ATHEROSCLEROSIS**
VERWENDUNG EINES BETABLOCKERS ZUR BEHANDLUNG VON ATHEROSKLEROSE
UTILISATION D'UN BETA-BLOQUANT POUR LE TRAITEMENT DE L'ATHEROSCLEROSE

(30) Priority: 22.06.2000 SE 0002354
(43) Date of publication of application: 02.04.2003
(73) Proprietor: AstraZeneca AB, 151 85 Södertälje (SE)
(72) Inventor: BERGLUND, Göran, S-217 74 Malmö (SE); WIKSTRAND, John, S-431 83 Mölndal (SE)
(74) Representative: Miller, Thomas Kerr
(86) International application number: PCT/SE2001/001380
(87) International publication number: WO 2001/097751

(56) References cited:
- WO-A1-00/38725
- WO-A1-97/38694
- WO-A1-98/02357
- WO-A1-99/11260
- G. BERGLUND ET AL.: '(MoW3:3) low dose metoprolol and fluvastatin slow progression of atherosclerosis: Main results from BCAPS' ATHEROSCLEROSIS vol. 151, no. 1, June 2000, page 4, XP002946310
- B. HEDBLAD ET AL.: '(MoP7:W3) low dose metoprolol and fluvastatin in silent atherosclerotic disease: Metabolic and adverse effects in BCAPS' ATHEROSCLEROSIS vol. 151, no. 1, June 2000, page 24, XP002946311
- GUDRUN WESTERGREN ET AL.: 'Effective once-daily treatment of hypertension with low-dose, controlled-release metoprolol' CURRENT THERAPEUTIC RESEARCH vol. 55, no. 2, February 1994, pages 142 - 148, XP002946312
- JOHN E. SANDERSON ET AL.: 'Effect of lowe dose beta blockers on atrial and ventricular (B type) natriuretic factor in heart failure: a double blind, randomised comparison of metoprolol and a third generation vasodilating beta blocker' BR. HEART J. vol. 74, 1995, pages 502 - 507, XP002946313
- C.J. EAGLES ET AL.: 'The effects of combined treatment with beta1-selective receptor antagonists and lipid-lowering drugs on fat metabolism and measures of fatique during moderate intensity exercise: a placebo-controlled study in healthy subjects' BR. J. CLIN. PHARMACOL. vol. 43, no. 3, 1997, pages 291 - 300, XP002946314
- OLAV PER FOSS ET AL.: 'Treatment of hypertensive and hypercholesterolaemic patients in general practice' SCAND. J. PRIM. HEALTH CARE vol. 17, 1999, pages 122 - 127, XP002946315
- JAMES L. POOL ET AL.: 'Lovastatin and coadministered antihypertensive/cardiovascular agents' HYPERTENSION vol. 19, no. 3, 1992, pages 242 - 248, XP002946316
- S. WITCHITZ, O. ET AL.: 'Effects compares du captopril et de l'atenolol sur le metabolisme lipidique chez des patients hypertendus hypercholesterolemiques traites par pravastatine' PRESSE MED. vol. 25, no. 40, December 1996, pages 2013 - 2016, XP002946317
- WILLIAM R. GARNETT: 'Interactions with hydroxymethylglutaryl-coenzyme A reductase inhibitors' AM. J. HEALTH-SYST. PHARM. vol. 52, August 1995, pages 1639 - 1645, XP002946318

## Description

The present invention relates to pharmaceutical formulations comprising a betablocker, in a maintenance dose lower than 50 mg, in admixture with a pharmaceutically acceptable adjuvant, diluent or carrier, as well as a method of treatment and use of the formulations for the treatment of atherosclerosis and related conditions.

There is a constant need for new medications to further reduce the risk of atherosclerotic disease, since this is one of the industrial world's most common health problems.

Various pharmaceuticals, such as the group known as betablockers, are known to have a positive influence on various cardiovascular diseases, whereas the effect upon atherosclerotic disease is little known. Betablockers have been shown to reduce cardiovascular events and mortality in secondary (The Norwegian Multicenter Study group, *N Engl JMed,* 304:501-807, 1981; Olsson et al., *J Am Coll Cardiol,* 5:1428-1437, 1985; MERIT-HF Study Group, *Lancet,* 353:2001-2007, 1999) and primary preventive studies (Wikstrand et al., *JAMA,* 259:1976-1982, 1988). In animal studies betablockers reduce the degree of diet-induced (Östlund-Lindqvist et al., *Arteriosclerosis,* 8;40-45, 1988) and stress-induced atherosclerosis (Kaplan et al., *Eur Heart J,* 8:928-944; Pettersson et al., *Curr Op in Cardiol* 3:S9-S14, 1988), but no direct evidence for an anti-atherosclerotic effect of beta-blockers, similar to the effects of statins on carotid artery intima-media thickness (IMT) have so far been shown in humans

The administration of combinations of β₁ selective blockers and lipid-lowering drugs to healthy volunteers in order to observe the effects on fat metabolism, ammonia levels and the perception of effort during exercise, was disclosed in Br. J. Clin Pharmacol 1997 vol 43, no 3 pages 291-300. The combinations studied were 1) metoprolol (controlled release) and fluvastatin 2) metoprolol (controlled release) and bezafibrate 3) atenolol (normal release) and fluvastatin and 4) atenolol (normal release) and bezafibrate. The paper concluded that that these four combinations each caused significant reductions in fat metabolism increased plasma ammonia concentrations and raised the perception of exercise. Combination 1) had the least adverse effect but the formulation difference was thought to be a significant factor in explaining the differences observed with respect to combination 3).

The effects of a combination of pravastatin and atenolol on hypertensive and hypercholesterolaemic patients were reported in Scand. J. Print Health Care 1999, vol 17 122-127. The conclusion was that the effect of atenolol was not influenced by the concurrent administration of pravastatin and *vice versa.* However, lifestyle intervention was also a feature of this study and therefore the conclusions which can be drawn from this study are not clear.

A *post hoc* analysis of patients who had been treated with lovastatin and who were also receiving antihypertensive medication including β₁ adrenergic receptor blockers was reported in Hypertension, 1992, Vol. 19,3 242. The paper concluded that, subject to a number of limitations, there was no evidence for an attenuation of the lovastsatin-induced changes in lipids and lipoproteins or an alteration in the safety profile of lovastatin when administered concurrently with commonly used antihypertensive agents.

It was concluded in Presse Med Volume 1996, vol 25, no.40, 2013-2016 that the effect of the β₁ adrenergic receptor blocker atenolol was not diminished in combination with pravastatin. However, the effect of pravastatin on lipid metabolism was more favourable when pravastatin was combined with the angiotensin converting enzyme inhibitor captopril rather than with atenolol.

Low doses of metoprolol for the treatment of hypertension and heart disease have been reported, but these reports indicate that the use of lower doses of metoprolol has a varying degree of efficacy. Westergren et al. in Current Therapeutic Research, 1994 vol. 55, No.2, 142, describe the use of a 50 mg dose of metoprolol (half of the most commonly used daily dose of immediate-release metoprolol) in the form of a controlled release tablet to treat mild-to-moderate hypertension. They report that this dose of metoprolol is well tolerated. However, Sanderson et al. allege in the British Heart Journal, 1995, vol. 74, 502, that a low dose of metoprolol of 6.25 mg administered twice daily can be hazardous in patients with severe heart failure.

A review by Garnett in Americal Journal of Health-System Pharmacology, 1995, vol. 52, 1639, describes the pharmacokinetic profiles of HMG-CoA reductase inhibitors and explores the specific drug interactions that have been documented.

WO 98/02357 discloses a carton for carrying pharmaceutically active substances or combinations thereof. One such combination mentioned is the combination of a beta blocker, such as metoprolol or isosorbidmononitrate, and a lipid lowering substance, such as fluvastatin. This application does not disclose any data concerning the effects of such a combination.

WO 99/11260 discloses a combination of atorvastatin and an antihypertensive agent. No data are disclosed in this application.

WO 97/38694 discloses a combination of an HMG-CoA reductase (3-hydroxy-3-methylglutaryl coenzyme A reductase) inhibitor in combination with folic acid in combination with a drug selected from a range of other types of drug including beta blockers.

WO 00/38725 discloses combinations of an ileal bile transport inhibitor and a range of other types of drug including antihypertensive drugs for example beta blockers. No data are presented.

The present invention relates to the use of a pharmaceutical formulation comprising a betablocker in a maintenance dose lower than 50 mg which is the highest dose of any betablocker that blocks beta-one receptors to a similar extent as 47 mg of metoprolol succinate in the reduction in exercise-induced heart rate increase over 24 hours in admixture with a pharmaceutically acceptable adjuvant, diluent or carrier in the manufacture of a medicament for the prophylactic or therapeutic treatment of atherosclerosis.

It has surprisingly been found that a low dose of a betablocker, especially metoprolol, can lower the rate of increase of carotid IMT in clinically healthy symptom-free subjects with a carotid plaque, which also indicates a favorable effect on atherosclerosis development.

In one aspect, the present invention relates to the use of a pharmaceutical formulation comprising a betablocker in a maintenance dose in the range of 25 - 47 mg in admixture with a pharmaceutically acceptable adjuvant, diluent or carrier. The dose of the betablocker is preferably lower than 30 mg, and most preferably 25 mg.

In the present application, the term "betablocker" refers to any pharmaceutical agent that as part of its pharmacological action blocks beta-one-receptors. Furthermore, in the present application, the term "betablocker" includes chemical modifications of betablockers such as esters, stereoisomers, prodrugs, and metabolites, whether active or inactive, and pharmaceutically acceptable salts or solvates of any of these, or solvates of such salts.

The betablockers referred to in this application include but are not limited to the compounds selected from the group consisting of acebutolol, alprenolol, amosulalol, arotinolol, atenolol, befunolol, betaxolol, bevantolol, bisoprolol, bopindolol, bucumolol, bufetolol, bufuralol, bunitrolol, buprandolol, butofilolol, carazolol, carteolol, carvedilol, celiprolol, cetamolol, cloranolol, dilevalol, epanolol, indenolol, labetalol, levobunolol, mepindolol, metipranolol, metoprolol, moprolol, nadolol, nadoxolol, nebivalol, nipradilol, oxprenolol, perbutolol, pindolol, practolol, pronethalol, propranolol, sotalol, sufinalol, talindol, tertatolol, tilisolol, timolol, toliprolol, and xibenolol, and stereoisomers thereof and pharmaceutically acceptable salts or solvates thereof, or solvates of such salts.

In the present invention the betablocker is suitably metoprolol or atenolol, and stereoisomers thereof, and pharmaceutically acceptable salts or solvates thereof, or solvates of such salts. Particularly, the betablocker is metoprolol succinate (disclosed in US 5,001,161), metoprolol tartrate or metoprolol fumarate.

Therefore, in another embodiment of this aspect of the invention, the betablocker is metoprolol or a pharmaceutically acceptable salt thereof, or a solvate of such a salt. Metoprolol may be in the form of metoprolol succinate, metoprolol fumarate, or metoprolol tartrate.

For clinical use, the betablocker is formulated into a pharmaceutical formulation for oral, intravenous, subcutaneous, tracheal, bronchial, intranasal, pulmonary, transdermal, buccal, rectal, parenteral or some other mode of administration. The pharmaceutical formulation contains the betablocker in admixture with a pharmaceutically acceptable adjuvant, diluent or carrier.

The total amount of active ingredient suitably is in the range of from about 0.1 % (w/w) to about 95 % (w/w) of the formulation, suitably from 0.5 % to 50 % (w/w) and particularly from 1 % to 25 % (w/w).

The pharmaceutical formulation can comprise a betablocker in a maintanance dose of less than 50mg and a cholesterol-lowering agent such as an HMG-CoA reductase inhibitor. The HMG-CoA reductase inhibitor may be a statin selected from atorvastatin, cerivastatin, fluvastatin, itavastatin, lovastatin, mevastatin, nicostatin, nivastatin, pravastatin and simvastatin or a pharmaceutically acceptable salt, especially sodium or calcium, or a solvate thereof, or a solvate of such a salt. An example of such a statin is a compound with the chemical name (E)-7[4-(4-fluorophenyl)-6-isopropyl-2-[methyl(methylsulfonyl)-amino]-pyrimidin-5-yl](3R,5S)-3,5-dihydroxyhept-6-enoic acid, and its calcium and sodium salts (disclosed in European Patent Application, Publication No. EP-A-0521471, and in Bioorganic and Medicinal Chemistry, (1997), 5(2), 437-444). The preferred beta blockers and the preferred doses of these beta blockers are as defined above.

The term "cholesterol-lowering agent" includes chemical modifications of the HMG-CoA reductase inhibitors such as esters, stereoisomers, prodrugs and metabolites, whether active or inactive. For the cholesterol-lowering agent, any dose used in clinical practice may be used in the formulations of the present invention.

The molar ratio between the betablocker and the cholesterol-lowering agent may be in the range of from about 1000:1 1 to 1:1000. The molar ratio between the betablocker and the cholesterol-lowering agent lies suitably in the range from 300:1 to 1:300, and particularly from 50:1 to 1:50.

In the preparation of the pharmaceutical formulations of the present invention the active ingredients may be mixed with solid, powdered ingredients, such as lactose, saccharose, sorbitol, mannitol, starch, amylopectin, cellulose derivatives, gelatin, or another suitable ingredient, as well as with disintegrating agents and lubricating agents such as magnesium stearate, calcium stearate, sodium stearyl fumarate and polyethylene glycol waxes. The mixture may then be processed into granules or pressed into tablets.

The active ingredients may be separately premixed with the other non-active ingredients, before mixed into a formulation. The active ingredients may also be mixed with each other, before being mixed with the non-active ingredients to form a formulation.

Soft gelatine capsules may be prepared with capsules containing the active ingredient of the invention, vegetable oil, fat, or other suitable vehicle for soft gelatine capsules. Hard gelatine capsules may contain granules of active ingredient. Hard gelatine capsules may also contain the active ingredient with combination with solid powdrered ingredients such as lactose, saccharose, sorbitol, mannitol, potato starch, corn starch, amylopectin, cellulose derivatives or gelatine.

Dosage units for rectal administration may be prepared (i) in the form of suppositories which contain the active substance mixed with a neutral fat base; (ii) in the form of a gelatine rectal capsule which contains the active substance in a mixture with a vegetable oil, paraffin oil or other suitable vehicle for gelatine rectal capsules; (iii) in the form of a ready-made enema; or (iv) in the form of a dry micro enema formulation to be reconstituted in a suitable solvent just prior to administration.

Liquid preparations may be prepared in the form of syrups or suspensions, e.g. solutions or suspensions containing the active ingredients and the remainder consisting, for example, of sugar or sugar alcohols and a mixture of ethanol, water, glycerol, propylene glycol and poyethylene glycol. If desired, such liquid preparations may contain coloring agents, flavoring agents, preservatives, saccharine and carboxymethyl cellulose or other thickening agents. Liquid preparations may also be prepared in the form of a dry powder to be reconstituted with a suitable solvent prior to use.

Solutions for parenteral administration may be prepared as a solution of a formulation of the invention in a pharmaceutically acceptable solvent. These solutions may also contain stabilizing ingredients, preservatives and/or buffering agents. Solutions for parenteral administration may also be prepared as a dry preparation to be reconstituted with a suitable solvent before use.

The dose of the compound to be administered will depend on the relevant indication, the age, weight and sex of the patient and may be determined by a physician. The dosage will particularly be in the range of from 0.01 mg/kg to 10 mg/kg, but the total daily dose will not exceed 50 mg.

### Medical and pharmaceutical use

Also provided according to the present invention is the use of a pharmaceutical formulation, comprising a betablocker, in a maintenance dose lower than 50 mg, particularly lower than 30mg and preferably in the range of 25 - 47 mg and particularly a dose of 25mg, in admixture with a pharmaceutically acceptable adjuvant, diluent or carrier, in the manufacture of medicaments for use in the prophylactic or therapeutic treatment of atherosclerosis comprising the administration of a therapeutically effective amount of a betablocker, in maintenance doses described immediately above, to a patient suffering from, or susceptible to, atherosclerosis.

The term "medical therapy" as used herein is intended to include prophylactic, diagnostic and therapeutic regimens carried out in vivo or ex vivo on humans or other mammals.

The formulations are expected to be useful in prophylactic or therapeutic treatment of atherosclerosis, particularly patients suffering from, or susceptible to coronary atherosclerosis, or carotid plaque.

The formulations are furthermore expected to be useful in prophylactic or therapeutic treatment of cardiovascular complications in general, including, but not limited to atherosclerosis including diet-induced and stress-induced atherosclerosis, cardiovascular death, hypertension, diabetes mellitus, angina pectoris, intermittent claudification, myocardial infarction, including acute myocardial infarction, and stroke.

More particularly the formulations are expected to be useful in prevention of clinical events associated with the progression of atherosclerosis and/or acute vascular accidents related to atherosclerotic disease and plaque including but not limited to stroke, myocardial infarction (MI), cognitive decline, peripheral vascular disease, and renal dysfunction.

The following example is intended to illustrate, but in no way limit the scope of the invention.

### EXAMPLE

A large scale clinical trial was designed to investigate the effect of low dose betablocker metoprolol on the progression of carotid IMT versus placebo and clinical outcome measures during 36 months double-blind treatment in asymptomatic subjects with a carotid plaque. The study was a randomized, double blind, parallel group, placebo-controlled, single-center study.

The study population consisted of asymptomatic men and women, aged 49 to 70 years, with a plaque in the right carotid artery. A random 50% of those who entered the study were invited to take part in a study on the epidemiology of carotid artery disease. 1548 subjects came to the enrolment examination (visit one) including medical history, physical examination, laboratory measurements and a two-dimensional B-mode ultrasound of the right carotid artery. Subjects with a qualifying lesion in the right carotid artery, and who had no contraindications to the study protocol were invited to take part. In all, 793 subjects were eligible for randomization. All participants provided written informed consent. Major exclusion criteria were history of myocardial infarction, angina pectoris or stroke within the preceding three months, history of surgical intervention in the right carotid artery, regular use of beta-blockers or statins, blood pressure above 160 systolic or 95 mm Hg diastolic, hypercholesterolemia (>8.0 mmol/L) , hyperglycemia requiring or suspected to require insulin treatment, and conditions which in the opinion of the investigator rendered the subject unsuitable for the trial.

The randomization procedure was performed by using a computer generated randomization scheme. Participants were, according to a factorial design, randomly assigned to one of four drug combination groups: placebo/placebo, metoprolol (25 mg o.d)/placebo, fluvastatin (40 mg o.d.)/placebo or metoprolol (25 mg o.d.)/ fluvastatin (40 mg o.d.). The placebo was manufactured to exactly resemble the metoprolol CR/XL tablets (AstraZeneca AB, Mölndal, Sweden), and fluvastatin (Novartis Ltd, Basel, Switzerland) capsules, respectively.

The primary outcome measures were the change in mean intima-media thickness (IMTmean) in the common carotid artery (10 mm long section), and the change in maximum intima-media thickness (IMTmax) in the carotid bulb. Safety and tolerability pattern during treatment with fluvastatin or metoprolol, was evaluated by comparing adverse events and laboratory findings to placebo. Death and incidence of major cardiovascular endpoints were monitored.

Every participant was seen four times during the first year (after one, three, six and twelve months) and every 6 months thereafter. Weight was obtained every six months and a fasting lipid profile (total cholesterol, LDL-lipoprotein, HDL-lipoprotein and triglycerides) was determined every year. Liver transaminases (AST, ALT) and creatine kinase (CK) were obtained at every visit during the first year and then every year thereafter. AST or ALT values ≥3 times, and CK values ≥10 times the upper limit of normal was considered elevated during the study. Carotid ultrasound investigation was performed at baseline and after 18 and 36 months treatment. Subjects with high serum cholesterol or triglycerides were advised to a low-fat diet and if evidence of persistent high cholesterol values such subjects were referred to an independent specialist of lipid disorders without knowledge of the subjects randomization assignment. Other conditions such as high blood pressure, congestive heart failure or abnormal laboratory values during the trial were dealt with in accordance with existing guidelines. At every visit each participant was asked about any hospitalization, acute myocardial infarction and stroke, since last visit. Vital status was obtained for all subjects at termination of the study.

Throughout the trial an Acuson 128 Computed Tomography System (Acuson, Mountain View, California) with a 7 MHz transducer was used. The examination procedure and image analysis, as described elsewhere (Wendelhag et al., *Clin Physiol,* 11:565-577, 1991; Wendelhag et al., *Stroke,* 28:2195-2200, 1997) was performed by specially trained sonographers certified upon completion of an extensive educational program (Berglund, et al., *J Intern Med,* 236:581-586, 1994). In brief, the right carotid bifurcation was scanned within a pre-defined window comprising three centimeters of the distal common carotid artery, the bifurcation and one centimeter of the internal and external carotid arteries, respectively, for the presence of plaques, defined as focal intima-media thickenings above 1.2 mm. Thickness of the intima-media complex was measured in the far wall according to the leading edge principle, using a specially designed computer-assisted image analyzing system based on automated detection of the echo structures, but with the option to make manual corrections by the operator. Each image was analyzed without knowledge of the subjects' randomization group.

From previous experience (Furberg et al., *Circulation,* 90:1679-1687, 1994) it was anticipated that the annual carotid artery IMTmean progression rate in the placebo group would be approximately 15 µm per year with a standard deviation of 30 µm. A sample size of 200 subjects per group, i.e. a total of 800 individuals, was determined based on a withdrawal rate of 20 %, a significance level of 5 % (two-sided), a power of 0.90, and a treatment effect of ≥ 75% (fluvastatin) and 30 % (metoprolol), respectively. The primary effect variables, change in IMTmean CCA, and IMTmax Bulb, was analyzed in a linear model with change in IMT as dependent variable and with treatment as a factor. Baseline IMTs and time between measurements were included in the model as covariates.

The randomization yielded well-balanced treatment groups, (see Table 1). Mean baseline LDL-cholesterol was 4.1 mmol/L. Current medication at baseline or during trial for other cardiovascular compounds (e.g. diuretics, calcium channel blockers, ACE-inhibitors, aspirin and postmenopausal hormone replacement therapy for women) were similar in the treatment groups (data not shown). The mean follow-up time was 35.9 (range 8 to 40) months.

### Metabolic and physiological effects of treatment

Metoprolol increased serum triglycerides by 0.14 mmol/L compared to the placebo group but no effect was observed on other metabolic variables. In comparison to the placebo group mean heart rate decreased in the metoprolol group by 2.5 beats per min, while blood pressure and lumen diameter were not significantly changed.

### Treatment effect on carotid IMT

Baseline ultrasound data are given in Table 1 and 2. The observed annual IMTmean CCA progression rate in the placebo group was 13 ±53 µm. The annual IMTmax progression rate in the bifurcation in the placebo group was 89±154 µm. Fluvastatin, but not metoprolol, reduced the rate of progression of IMTmean CCA compared with placebo after 36 months of treatment (mean difference of yearly change between groups: -9, 95% confidence interval [CI]: -15 to -3 µm, p=0.002), Table 2-3.

Metoprolol was effective in slowing the progression rate of carotid IMTmaxBulb compared with placebo after 36 months of treatment (mean difference of yearly change between groups: -23, 95% CI: -44 to -3 µm, p=0.014), Table 2-3. This effect was evident already after 18 months' of treatment (mean difference of yearly change between groups: -58, 95% CI: -94 to -23 µm, p=0.004). Metoprolol CR/XL also reduced the progression rate of IMTmaxBulb after 36 months of treatment in the subgroup with serum cholesterol ≥6.5 mmol/L at baseline (mean difference of yearly change between groups: -53, 95% CI: -87 to -19 µm, p=0.001).

### Incidence of cardiovascular events during follow-up

Eighteen of the participants had a cardiovascular (CVD) event (one subject suffered a fatal and seven a non-fatal myocardial infarction, two died suddenly because of ischaemic heart disease, and eight suffered a non-fatal stroke). The CVD event rate tended to be lower in the metoprolol group compared to the placebo group (5 versus 13 cases, p=0.055). Corresponding numbers in the fluvastatin and placebo groups were 7 and 11 cases, respectively, p=0.350.

### Tolerability

Permanent withdrawal from randomized treatment was 15% in the metoprolol group, 21 % in the fluvastatin group, and 23% in the placebo group. The withdrawal rate in the group of the combination of the two drugs was 25 %. Women in the fluvastatin group had, in comparison with the placebo group, an increased frequency of transiently high liver enzymes (10.2 % versus 1.8 %). Incidence of severe adverse events or cancer did not differ between the treatment groups.

**TABLE 1.**

| **Characteristics** | | **Treatment group** | | |
|---|---|---|---|---|
| | | Placebo/ Placebo | Metoprolol/ Placebo | Metoprolol/ Fluvastatin |
| Sex | | | | |
| | Men, n (%) | 92 (46.2) | 89 (44.7) | 86 (43.7) |
| | Women, n (%) | 107 (53.8) | 110 (55.3) | 111 (56.3) |
| Age (years) | | 61.9 ± 5.4 | 61.1 ± 5.6 | 62.2 ± 5.2 |
| Current smokers (%) | | 57 (28.6) | 63 (31.7) | 66 (33.5) |
| Body mass index (kg /m2) | | 25.6 ± 3.7 | 25.6 ± 3.8 | 25.4 ± 3.4 |
| Systolic blood pressure (mm Hg) | | 139.1 ±14.6 | 137.9 ± 15.0 | 139.1 ± 13.7 |
| Diastolic blood pressure (mm Hg) | | 84.5 ± 8.0 | 84.5 ± 6.9 | 84.8 ± 6.6 |
| Heart rate (beats/min) | | 69.5 ± 9.0 | 68.9 ± 8.4 | 70.2 ± 9.0 |
| History of hypertension, n (%) | | 22(11.1) | 21 (10.6) | 24 (12.2) |
| Serum cholesterol (mmol/L) | | 6.05 ± 0.98 | 6.14 ± 1.03 | 6.14 ± 0.93 |
| | ≥ 6.5 mmol /L, n (%) | 66 (33.2) | 70 (35.4) | 68 (34.7) |
| LDL(mmol/L) | | 4.1 ± 0.9 | 4.2 ± 1.0 | 4.2 ± 0.9 |
| HDL (mmol /L) | | 1.40 ± 0.41 | 1.35 ± 0.37 | 1.39 ± 0.35 |
| Triglycerides (mmol /L) | | 1.21 (0.48-3.57) | 1.18 (0.46-4.57) | 1.12 (0.41 - 4.61) |
| History of hyperlipidemia, n (%) | | 31 (15.6) | 42 (21.1) | 37 (18.8) |
| Fasting blood glucose (mmol/L) | | 5.1 ± 0.9 | 5.2 ± 0.7 | 5.1 ± 0.6 |
| History of NIDDM, n (%) | | 10 (5.0) | 7 (3.5) | 5(2.5) |
| History of CVD, n (%) | | 7 (3.5) | 8 (4.0) | 8 (4.1) |

| Carotid ultrasonography | | | | |
|---|---|---|---|---|
| | Common carotid IMTmean (µm) | 898 ± 171 | 920 ± 197 | 903 ± 205 |
| | Bifurcation carotid IMTmax Bulb (µm) | 1875 ± 505 | 1936 ± 651 | 1970 ± 652 |

Selected baseline characteristics of randomized BCAPS participants by treatment group. Abbreviations: LDL, low density lipoprotein; HDL, high density lipoprotein; NIDDM, non-insulin dependent diabetes mellitus; CVD, cardiovascular disease; IMT, intima-media thickness.

**TABLE 2.**

| **Characteristics** | | **Treatment groups** | | | |
|---|---|---|---|---|---|
| | | Placebo | Metoprolol | Placebo | Fluvastatin |
| Common carotid IMTₘₑₐₙ (µm) | | | | | |
| | Number of subjects | 390 | 393 | 394 | 389 |
| | Baseline (± SD) | 893 ± 170 | 912 ± 202 | 910 ± 186 | 895 ± 188 |
| | 18 months (± SD) | 896 ± 176 | 908 ± 205 | 913 ± 186 | 890 ± 196 |
| | 36 months (± SD) | 917 ± 203 | 934 ± 220 | 945 ± 216 | 905 ± 205 |
| | Δ 18 months to baseline (± SD) | 3 ± 10 | -5 ± 12 | 3 ± 11 | -5 ± 11 |
| | Δ 36 months to baseline (± SD) | 24 ± 13 | 22 ± 13 | 36 ± 15 | 11 ± 11 |
| | Δ between groups at 36 months (95% CI) | | -2 (-20 to 17) | | -25 (-44 to -7) |
| | | | | | |

| Bifurcation carotid IMTₘₑₐₙ (µm) | | | | | |
|---|---|---|---|---|---|
| | Number of subjects | 369 | 364 | 375 | 358 |
| | Baseline (± SD) | 1875 ± 541 | 1936 ± 634 | 1886 ± 570 | 1926 ± 609 |
| | 18 months (± SD) | 1982 ± 572 | 1937 ± 572 | 1954 ± 589 | 1965 ± 554 |
| | 36 months (± SD) | 2133 ± 630 | 2003 ± 624 | 2097 ± 670 | 2095 ± 652 |
| | Δ 18 months to baseline (± SD) | 112 ± 376 | 23 ± 325 | 72 ± 373 | 63 ± 334 |
| | Δ 36 months to baseline (± SD) | 227 ± 421 | 154 ± 411 | 211 ± 455 | 170 ± 374 |
| | Δ between groups at 36 months (95% CI) | | -73 (-133 to - 13) | | -41 (-102 to 19) |

Mean values for and mean change in common and bifurcation carotid intima-media thickness at baseline and at the 18 and 36 months follow-up according to treatment groups. IMT, intima-media thickness; SD, standard deviation; CI, confidence interval.

**TABLE 3.**

| **Outcome measure** | **Treatment** | | **β-coefficient** | **Standard error** | **p-value** |
|---|---|---|---|---|---|
| IMTₘₑₐₙ CCA | | | | | |
| | Fluvastatin | | | | |
| | | 18 months | -0.0135 | 0.008 | 0.077 |
| | | 36 months | -0.0275 | 0.009 | 0.002 |

| | Metoprolol | | | | |
|---|---|---|---|---|---|
| | | 18 months | 0.0049 | 0.008 | 0.524 |
| | | 36 months | 0.0012 | 0.009 | 0.897 |
| | | | | | |

| IMTₘₐₓ bifurcation | | | | | |
|---|---|---|---|---|---|
| | Fluvastatin | | | | |
| | | 18 months | 0.0009 | 0.029 | 0.940 |
| | | 36 months | -0.0132 | 0.013 | 0.329 |

| | Metoprolol | | | | |
|---|---|---|---|---|---|
| | | 18 months | -0.0367 | 0.013 | 0.004 |
| | | 36 months | -0.0333 | 0.013 | 0.014 |

Treatment effects during 36 months on progression (in µm) of the common carotid artery IMTmean and the carotid artery bifurcation IMTmax, respectively. Intention-to-treat analysis. Adjusted for baseline IMTmean and IMTmax, respectively, and time between measurements

## Claims

1. The use of a pharmaceutical formulation comprising a betablocker in a maintenance dose lower than 50 mg which is the highest dose of any betablocker that blocks beta-one receptors to a similar extent as 47 mg of metoprolol succinate in the reduction in exercise-induced heart rate increase over 24 hours in admixture with a pharmaceutically acceptable adjuvant, diluent or carrier in the manufacture of a medicament for the prophylactic or therapeutic treatment of atherosclerosis.

2. The use according to claim 1 comprising a betablocker in a maintenance dose in the range of 25 - 47 mg in admixture with a pharmaceutically acceptable adjuvant, diluent or carrier.

3. The use according to according to claim 2 wherein the dose of the betablocker is 25 mg.

4. The use according to any previous claim wherein the betablocker is metoprolol or stereoisomers thereof, or a pharmaceutically acceptable salt thereof, or a solvate of such a salt,

5. The use according to claim 4 wherein the betablocker is metoprolol succinate, metoprolol fumarate, or metoprolol tartrate,

6. The use according to claim 5 wherein the betablocker is metoprolol succinate.

## Patentansprüche

1. Verwendung einer pharmazeutischen Formulierung, enthaltend einen Betablocker in einer Erhaltungsdosis von unter 50 mg, der Höchstdosis an Betablocker zur Blockierung von Beta-1-Rezeptoren in ähnlichem Ausmaß wie 47 mg Metoprololsuccinat bei der Reduzierung des belastungsinduzierten Herzfrequenzanstiegs über 24 Stunden, dem ein pharmazeutisch annehmbares Adjuvans, Verdünnungsmittel oder Trägermedium beigemischt ist, zur Herstellung eines Arzneimittels zur prophylaktischen oder therapeutischen Behandlung von Atherosklerose.

2. Verwendung nach Anspruch 1 eines Betablockers in einer Erhaltungsdosis im Bereich von 25-47 mg, dem ein pharmazeutisch annehmbares Adjuvans, Verdünnungsmittel oder Trägermedium beigemischt ist.

3. Verwendung nach Anspruch 2, wobei die Betablocker-Dosis 25 mg beträgt.

4. Verwendung nach einem der vorangehenden Ansprüche, wobei es sich bei dem Betablocker um Metoprolol oder Stereoisomere davon, oder um ein pharmazeutisch annehmbares Salz davon oder ein Solvat eines derartigen Salzes handelt.

5. Verwendung nach Anspruch 4, wobei es sich bei dem Betablocker um Metoprololsuccinat, Metoprololfumarat oder Metoprololtartrat handelt.

6. Verwendung nach Anspruch 5, wobei es sich bei dem Betablocker um Metoprololsuccinat handelt.

## Revendications

1. Utilisation d'une formulation pharmaceutique comprenant un bêta-bloquant dans une dose d'entretien inférieure à 50 mg, ce qui représente la dose la plus élevée de tout bêta-bloquant qui bloque des récepteurs bêta 1 d'une manière semblable à 47 mg de succinate de métoprolol dans la réduction de l'augmentation du rythme cardiaque induit par de l'exercice sur une période de 24 heures, en mélange avec un adjuvant, un diluant ou un excipient pharmaceutiquement acceptable dans la fabrication d'un médicament pour le traitement prophylactique ou thérapeutique de l'athérosclérose.

2. Utilisation selon la revendication 1 comprenant un bêta-bloquant dans une dose d'entretien dans la plage de 25 à 47 mg en mélange avec un adjuvant, un diluant ou un excipient pharmaceutiquement acceptable.

3. Utilisation selon la revendication 2, dans laquelle la dose du bêta-bloquant est de 25 mg.

4. Utilisation selon l'une quelconque des revendications précédentes, dans laquelle le bêta-bloquant est du métoprolol ou des stéréoisomères de celui-ci, ou un sel pharmaceutiquement acceptable de celui-ci ou un solvate d'un tel sel.

5. Utilisation selon la revendication 4, dans laquelle le bêta-bloquant est du succinate de métoprolol, du fumarate de métoprolol ou du tartrate de métoprolol.

6. Utilisation selon la revendication 5, dans laquelle le bêta-bloquant est du succinate de métoprolol.
